# EUROPEAN PATENT APPLICATION

(11) **EP 2 738 591 A1**
(43) Date of publication of application: **04.06.2014**
(21) Application number: 12194692.5
(22) Date of filing: 28.11.2012
(51) Int. Cl.: G02C 5/00, A61F 9/02

(54) **Sliding lens spectacles**

(71) Applicant: Al-Basri, Akeel, Prestwich Manchester Greater Manchester M25 0AD (GB)
(72) Inventor: Al-Basri, Qasim, Manchester, Greater Manchester M25 0AD (GB)

(57) **Abstract**

This invention is a spectacles device comprising four sliding lenses (1-4) to aid the user to change lenses swiftly at the front of the visual position, each two lenses are connected by a cord, and the sliding occurs when the sliders are moved in opposite directions.

## Description

### Background;

This invention relates to a new device designed specifically for spectacles to house multiple lenses within the spectacle frame with a control mechanism for the wearer to easily slide and change the lenses in a single movement, from the lens currently in their line of vision to an alternate lens housed in the spectacle frame.

### Statement of invention;

The Sliding lens spectacle invention combines multiple lenses to be used in one single spectacle frame with the ability for the wearer to control the lenses positioned in the line of vision, to different lenses suitable to the user needs at a point in time.

### Advantages;

The Sliding lens spectacles invention combines multiple lenses to be used in one single spectacle frame. This combination of lenses will eliminate the need for using two or more spectacles of different distance vision, or the need to have reading glasses and long distance glasses or reading glasses and 3D TV glasses or reading glasses with sun glasses or reading glasses with welding glasses or indeed any other variation of vision glasses. The benefit of combining and merging multiple types of lenses in one spectacle frame will speed up the changing of vision aid as needed compared with the wearer continually having to swap between different pairs of spectacles.

### Introduction to Drawings;

The description of the inventive Sliding lens spectacles (SLS) is illustrated in the drawings attached.

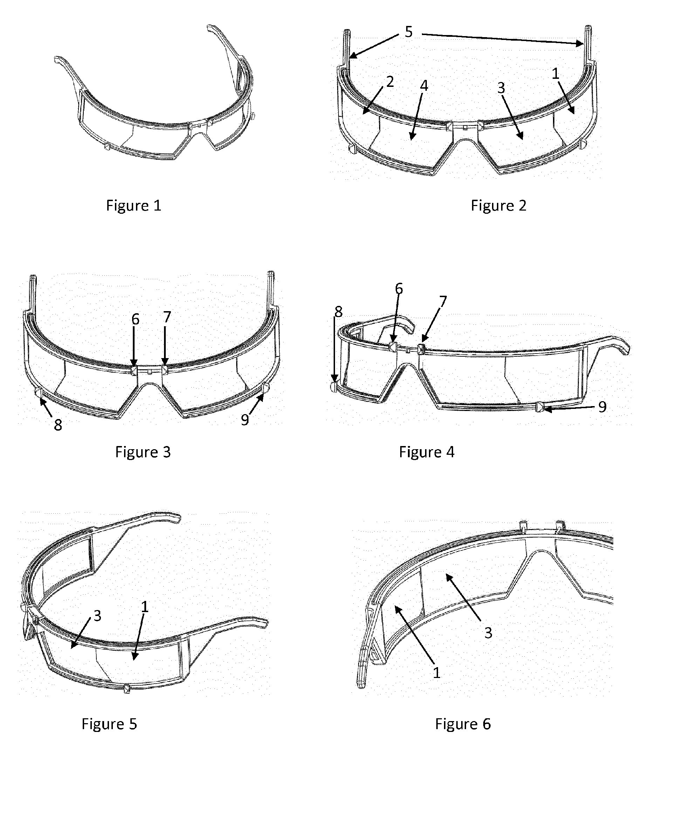

Figure 1: Shows isometric view of the SLS.
Figure 2: Shows the SLS from another angle, Left Front Lens (1), Right Front Lens (2), Left Rear Lens (3), Right Rear Lens (4) and Frame (5).
Figure 3: Shows the SLS from another angle, Right Upper Slider (6), Left Upper Slider (7), Right Lower Slider (8), Left Lower Slider (9).
Figure 4: Shows the SLS from another angle, Right Upper Slider (6), Left Upper Slider (7), Right Lower Slider (8).
Figure 5: Shows the SLS from another angle, Left Front Lens (1), Left Rear Lens (3).
Figure 6: Shows the SLS from another angle, Left Front Lens (1), Left Rear Lens (3).

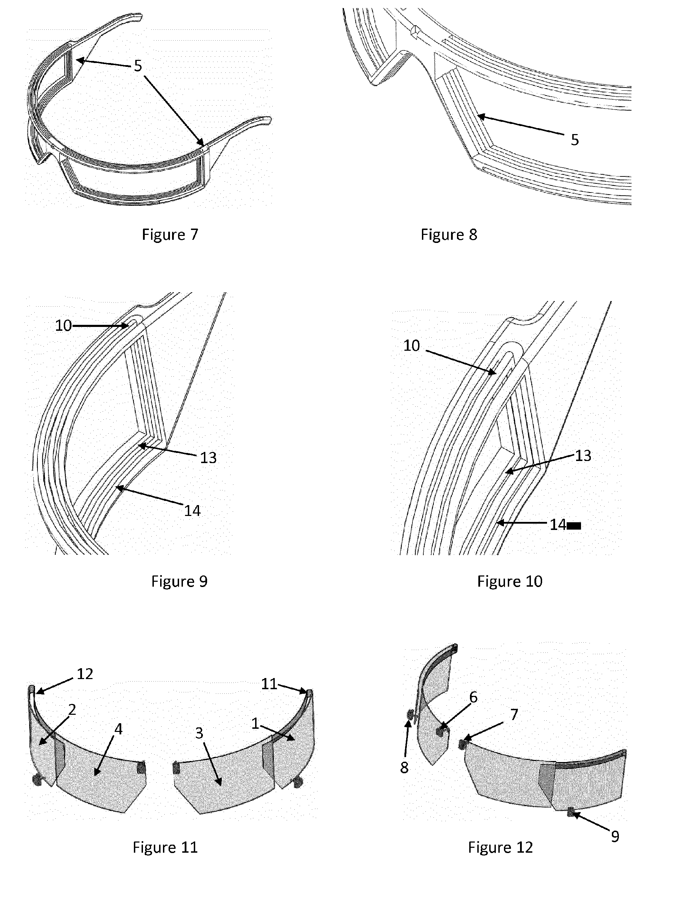

Figure 7: Shows the SLS frame (5).
Figure 8: Shows enlarged view of the SLS frame.
Figure 9: Shows a view of the SLS frame comprising the two tracks, the Front Track (13), the Rear Track (14), and the Track Separator (10).
Figure 10: Shows enlarged view of the SLS frame comprising the two tracks, the Front Track (13), the Rear Track (14), and the Track Separator (10).
Figure 11: Show isometric view of the SLS lenses parts without the frame, Left Front Lens (1), Right Front Lens (2), Left Rear Lens (3) and Right Front Lens (4), attached by the Right Cord (12), and the Left Cord (11).
Figure 12: Show another view of the SLS lenses with the Sliders attachments, Right Upper Slider (6), Left Upper Slider (7), Right Lower Slider (8), Left Lower Slider (9).

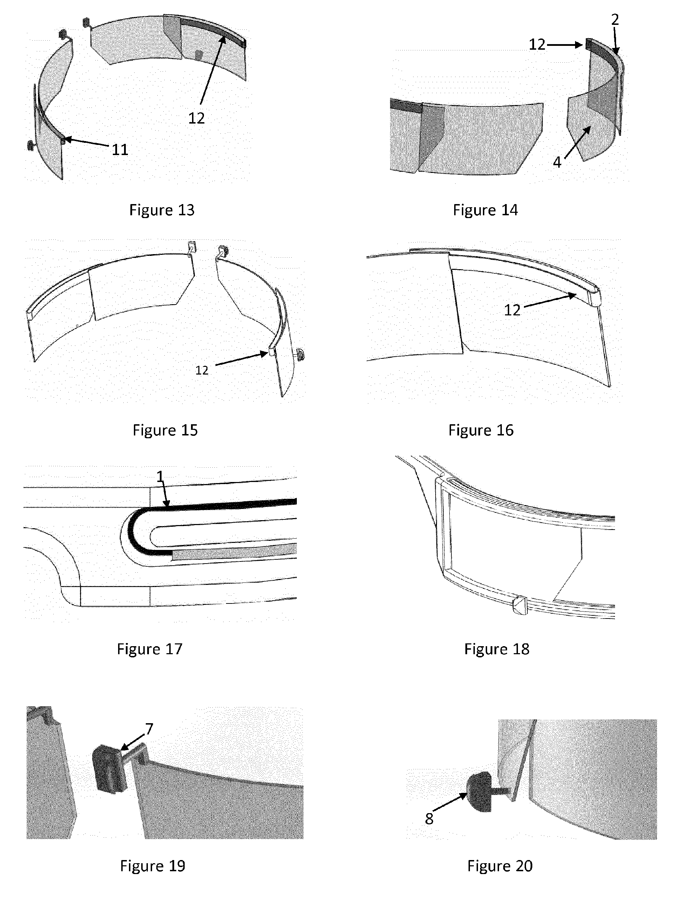

Figure 13: Show another view of the SLS lenses with the cords attachments, the Left Cord (11), the Right Cord (12).
Figure 14: is enlarged view of the SLS lenses with the Right Cord (12), Right Front Lens (2), Right Rear Lens (4).
Figure 15: is a view of the SLS lenses with the Right Cord (12).
Figure 16: is another view of the SLS lenses with the Right Cord (12).
Figure 17: is enlarged plan view of the SLS frame showing the Right Cord (11).
Figure 18: is enlarged close view of the SLS frame.
Figure 19: Show close enlarge view of the Left Upper Slider (7).
Figure 20: Show close enlarge view of the Right Lower Slider (8).

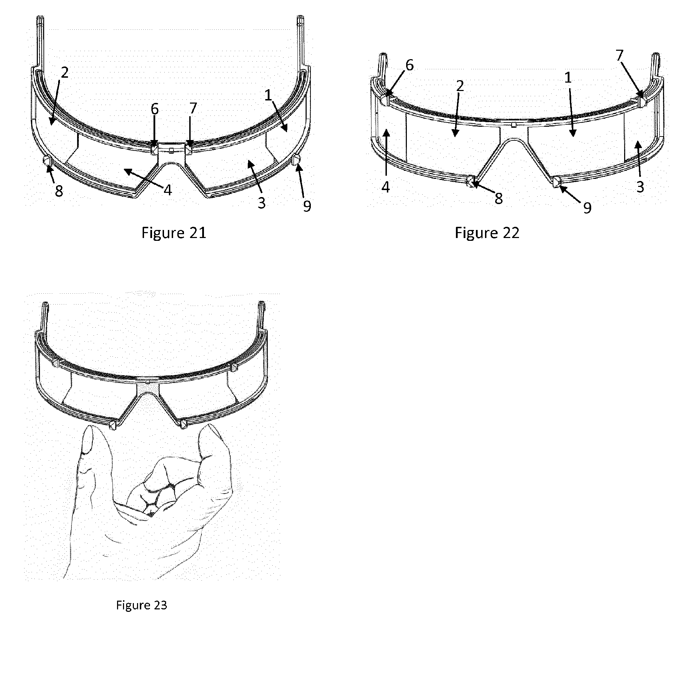

Figure 21: Shows view of the SLS, with the Sliders in a position of the Right Upper Slider (6), Left Upper Slider (7) are close to the middle securing the Left Rear Lens (3) and Right Rear Lens (4) on the middle visual position, and the Right Lower Slider (8), Left Lower Slider (9) are far apart, and Left Front Lens (1), Right Front Lens (2), far apart outside of the middle visual position.
Figure 22: Shows view of the SLS, with the siders in a position that the Right Upper Slider (6), Left Upper Slider (7) are far apart, and the Right Lower Slider (8), Left Lower Slider (9) are close to the middle, securing the Left Front Lens (3) and Right Front Lens (4) far apart outside of the middle visual position, and Left Rear Lens (1), Right Rear Lens (2), in the middle at the visual position.
Figure 23: Shows view of the SLS with hand positioned ready to operate the sliders.

The Sliding lens spectacle invention combines multiple lenses to be used in one single spectacle frame with the ability for the wearer to control the lenses positioned in the line of vision, to different lenses suitable to the user needs at a point in time. Each two lenses are connected by a cord and controlled by manually moving the sliders. Moving any of the lenses by the attached sliders in one direction will result in pulling the connected lens via the cord in the opposite direction.

The device consist of four lenses, Right Rear Lens, Left Rear Lens, Right Front Lens, and Left Front Lens, each lens is attached to a slider, and these sliders are Left Upper Slider, Right Upper Slider, Left Lower Slider, and Right Lower Slider, the lenses are attached by two cords, the Left Cord connecting the Left Rear Lens to Left Front Lens, and The Right Cord connecting the Right Rear Lens to the Right Front Lens, so any movement to any lens will affect the other connected lens. The frame of the spectacles consist of two tracks the front track housing the Right Front Lens and Left Front Lens, and the back track is housing the Right Rear Lens and Left Rear Lens and the tracks are separated by a separator.

When the Upper Sliders are close to each other it will result in the Front Lenses being far apart from each other at the same time the Rear Lenses will be close to each other at the visual path position.

When the Lower Sliders are close to each other it will result in the Rear Lenses being far apart from each other at the same time the Front Lenses will be close to each other at the visual path position.

To operate the sliding mechanism a force is required to push the Upper Sliders or the Lower Sliders close to each other. If force applied to the Lower Sliders to be pushed close to each other, a sliding movement will occur to all lenses due to the linkage of the Cords to the Lenses, and this movement will result in positioning the Right Front Lens and Left Front Lens to be at the visual position at the same time the Right Rear Lens and Left Rear Lens will be far apart outside the visual position resulting in change of position of the Left Upper Slider and the Right Upper Slider being far apart.

If force applied to Left Upper Slider and Right Upper Slider to move close to the middle this will result in repositioning all sliders and lenses back to the original positions, and by applying force to the Upper and Lower Sliders to be close to the middle all lenses will change position according to the user needs and vice versa.

## Claims

1. A spectacles assembly comprising a number of parts, those being a Right Rear Lens, a Left Rear Lens, a Right Front Lens, and a Left Front Lens, each lens being attached to a slider, these sliders being Left Upper Slider, Right Upper Slider, Left Lower Slider, and Right Lower Slider, the lenses being attached by two cords, the Left Cord connecting the Left Rear Lens to Left Front Lens, and the Right Cord connecting the Right Rear Lens to the Right Front Lens, where any movement to any lens affects the other connected lens, the frame of the spectacles consisting of two tracks, the front track housing the Right Front Lens and Left Front Lens, and the back track housing the Right Rear Lens and Left Rear Lens.

2. A spectacles assembly as claimed in claim 1, designed to function on sliding mechanism to change vision aid by swapping lenses.

3. A spectacles assembly claimed in any preceding claims designed to function with any alternative manual mechanisms enabling and resulting in the sliding movement of the lenses.

4. A spectacles assembly claimed in any preceding claims designed with any form of automated, motorised or electronic control for moving and sliding the lenses.

5. A spectacles assembly claimed in any preceding claims designed with any form of automated, motorised or electronic control with electronic sensors controlling the sliding of the lenses by movement detection.

6. A spectacles assembly claimed in any preceding claims designed to function using a frame made of any material such as plastic, metal, titanium or similar.

7. A spectacles assembly claimed in any preceding claims designed to function using any type of lenses such as prescription lenses, sun lenses, 3D lenses, welding lenses, magnifying lenses, or any lenses made of any material.

8. A spectacles assembly claimed in any preceding claims housing more than two lenses.
